# EUROPEAN PATENT SPECIFICATION

(11) **EP 0 421 280 B1**
(45) Date of publication and mention of the grant of the patent: **12.01.1994**
(21) Application number: 90118591.8
(22) Date of filing: 27.09.1990
(51) Int. Cl.: F04D 11/00, F04D 33/00, A61M 1/10

(54) **Precessing centrifugal pump integrally combined with a motor**
Präzessionspumpe mit komplett eingebautem Motor
Pompe à précession intégralement combinée avec un moteur

(30) Priority: 30.09.1989 JP 256386/89
(43) Date of publication of application: 10.04.1991
(73) Proprietor: UBE INDUSTRIES, LTD., Ube-shi, Yamaguchi-ken 755 (JP)
(72) Inventor: Akamatsu, Teruaki, Room 208, Kamigyo-ku, Kyoto-shi, Kyoto 602 (JP)
(74) Representative: Schwabe - Sandmair - Marx

(56) References cited:
- EP-A- 0 060 569
- WO-A-83/00725
- WO-A-86/04962

## Description

This invention relates to a precessing centrifugal pump integrally combined with a motor and, more particularly, to a precessing centrifugal pump integrally combined with a motor and having a precessing impeller for rotating and pumping out a fluid.

Recently, trials of applying a small light centrifugal pump as a blood pump to clinical treatment have become noticeable. However, a small centrifugal pump simply designed based on the ordinary centrifugal pump is unusable because it entails the risk of bacteria or extraneous substances entering the pump through the bearings or the sealing portions and the risk of hemolysis (hemocytocatheresis). A precessing centrifugal pump has been proposed to solve these problems which has an impeller structure in which an impeller (head) is attached to a precessing rod instead of a rotating shaft. The inventor of the present invention has also studied in various ways for the purpose of improving the efficiency of this type of centrifugal pump and has filed an international application. (Refer to International Publication WO 86/04962.)

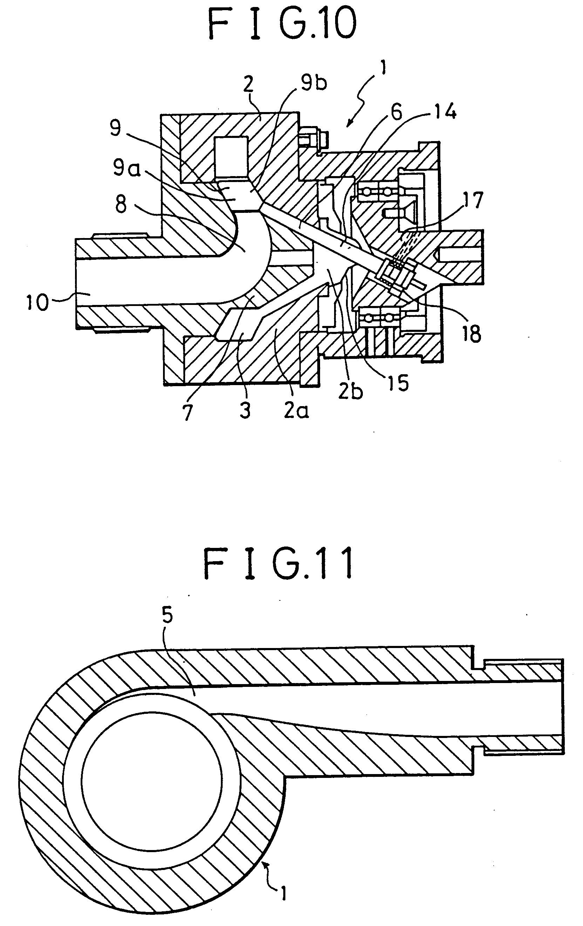

This international application discloses a centrifugal pump, such as that shown in Figs. 10 and 11, which has been provided as a precessing centrifugal blood pump having an improved pumping efficiency and capable of effectively preventing hemolysis.

Referring to Figs. 10 and 11, a centrifugal pump 1 has an annular chamber 3 formed in a casing 2 with a conical internal space. The casing 2 has a side wall 2a and an opening 2b formed at the apex the conical shape. The annular chamber 3 is defined by the side wall of the casing 2 at the outer peripheral side and is defined by a flow guide wall 7 at the inner peripheral side. The flow guide wall 7 projects from the center of a circular end surface of the casing 2 into the annular chamber 3.

The annular chamber 3 communicates through an inlet passage 8 with an inlet 10 opened at the center of the circular end surface of the casing 2. The inlet passage 8 is a spiral flow passage formed through the flow guide wall 7. The annular chamber 3 communicates with an outlet 5 which extends tangentially from a portion of the side wall 2a of the casing 2 facing the inlet passage 8 (and smoothly expanded outwardly).

An impeller 6 having a rod 14 and a head 9 consisting of a conical end portion 9a and a base portion 9b is movably set in the annular chamber 3. The impeller 6 extends along the side wall 2a of the casing 2 and the flow guide wall 7 in the annular chamber 3. The rod 14 extends out of the annular chamber 3 through the opening 2b of the casing 2.

The opening 2b is covered with a sealing membrane 15 formed of a material flexible and having sufficient durability against repeated bending, e.g., polyurethane or silicone. The sealing membrane 15 is attached to the side wall 2a of the casing 2 and to the impeller 6 in a water-tight manner. The rod 14 of the impeller 6 extending out of the annular chamber 3 is connected to a support 17 through a bearing 18. The support 17 when rotated creates a precessing motion of the impeller 6 with the precession center located at the opening 2b. The support 17 is connected to a motor shaft (not shown) and is thereby rotated.

This precessing centrifugal pump is capable of limiting disturbance or the like of the flow in the pumping chamber to a certain extent, and the pumping efficiency of this pump is high, about 40 %.

However, further improvements in the pumping performance and the effect of preventing hemolysis are expected, and this precessing centrifugal pump is also unsatisfactory because it is not integrally combined with the motor; the support 17 (rotary member) on which the rod 14 of the impeller 6 is supported is connected to the motor at its right end as viewed in Fig. 10.

### SUMMARY OF THE INVENTION

The inventor of the present invention has further studied and examined the shapes of the pump inlet, the pump outlet and the impeller head as well as a structure for integrally combining the centrifugal pump and the motor, and has thereby achieved the present invention.

According to the present invention, there is provided a precessing centrifugal pump characterized by comprising:
a centrifugal pump unit including an annular chamber formed in a generally conical casing and defined by a side wall of the casing at the outer peripheral side, an impeller having a head and a rod movably accommodated in the annular chamber, a flow guide wall projecting from the center of a circular end surface of the casing into the annular chamber to define same at the inner peripheral side thereof, an inlet passage formed in the flow guide wall, an inlet communicating with the annular chamber through the inlet passage and opening at the center of the circular end surface of the casing, an outlet extending tangentially from the annular chamber, the rod of the impeller extending through an opening of the casing formed at the apex thereof, and a sealing membrane covering the opening of the casing and attached in a water-tight manner to a side wall of the casing and to the impeller, the inlet passage being formed so as to have an spiral opening extending from a projecting end portion of the flow guide wall through the whole length thereof; and
a motor unit including a rotary member to which the rod of the impeller is attached and fixed, a rotor having a magnet attached to an outer circumferential surface of the rotary member and rotated together with the rotary member, and a coil disposed so as to face the magnet; the motor unit being integrally combined with the centrifugal pump unit.

This precessing centrifugal pump is constructed by integrally combining the centrifugal pump unit and the motor unit, can therefore be directly embedded in a human body, and is very conveniently used for a pump-oxygenator. Because the inlet passage being formed so as to have an spiral opening extending from a projecting end portion of the flow guide wall through the whole length thereof, the fluid inflow area is increased and the pumping effect of the rod of the impeller can also be utilized.

Also, in the precessing centrifugal pump in accordance with the present invention, an outer peripheral portion of the outlet extending tangentially from the annular chamber may be slightly projected in a direction opposite to the flow whirling direction to prevent a vortex flow occurring at this position by mixing of an outer peripheral flow having high potential energy (having a larger whirling radius) as well as to make the flows uniform.

Further, in this precessing centrifugal pump, the head of the impeller may be formed into a club-like shape to apply suitable whirling energy to the fluid flowing into the pump.

### BRIEF DESCRIPTION OF THE DRAWINGS

The stated object and other features of the present invention will be better be understood by referring to the description of the embodiments of the present invention taken in conjunction with the accompanying drawings in which:
Fig. 1 is a cross-sectional view of a precessing centrifugal pump integrally combined with a motor in accordance with an embodiment of the present invention;
Fig. 2 is a cross-sectional view of another embodiment of the present invention;
Figs. 3 to 5 are schematic diagrams of arrangements in each of which two centrifugal pump units are connected to opposite sides of a motor unit;
Figs. 6(a)A to 6(f) are diagrams of an example of the centrifugal pump unit;

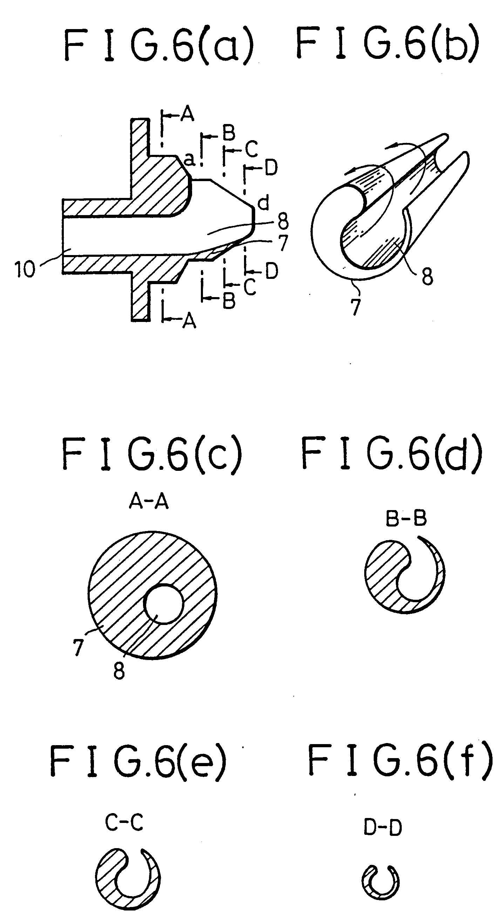

Fig. 6(a) is a cross-sectional view of an inlet portion;
Fig. 6(b) is a perspective view of an inlet passage;
Fig. 6(c) is a cross-sectional view taken alone the line A - A of Fig. 6(a);
Figs. 6(d) to (f) are cross-sectional views taken alone the line A - A, the line B - B, the line C - C, and the line D - D of Fig. 6(a), respectively;

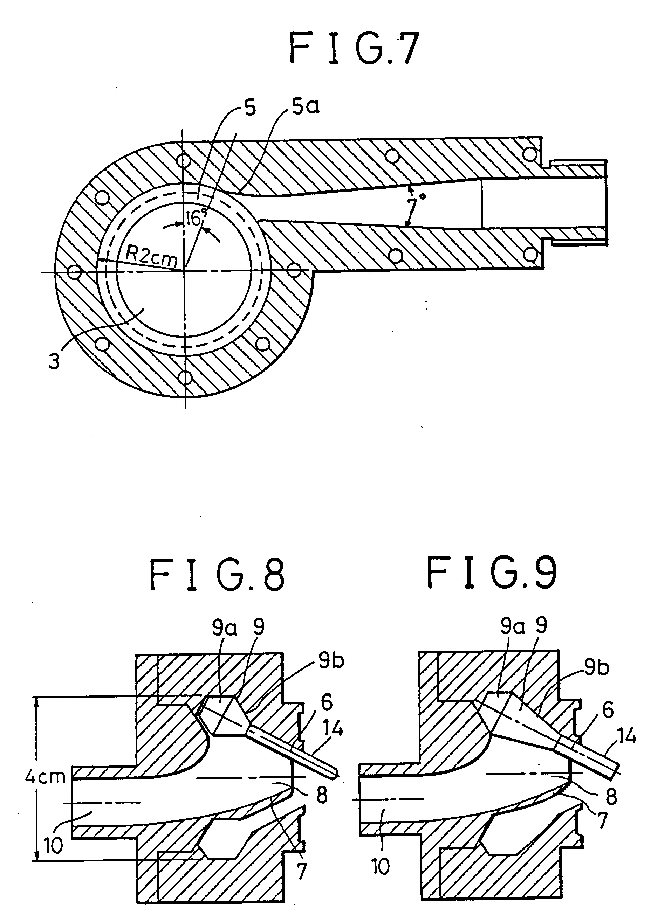

Fig. 7 is a transverse cross-sectional view of the centrifugal pump unit, showing the shape of the outlet;
Figs. 8 and 9 are axial cross-sectional views of other examples of the centrifugal pump unit; and
Figs. 10 and 11 are axial and transverse cross-sectional views of a conventional precessing centrifugal pump.

### DESCRIPTION OF THE PREFERRED EMBODIMENTS

The present invention will be described below in detail with respect to illustrated embodiments thereof but the invention is not limited to these embodiments.

For ease of description, the embodiments of the present invention will be compared with the conventional pump structure shown in Figs. 10 and 11.

Of a precessing centrifugal pump integrally combined with a motor in accordance with the present invention, a centrifugal pump unit I will be first described below.

Figs. 6(a) to 6(f) show an example of the centrifugal pump unit of the precessing centrifugal pump of the present invention. The structure of an inlet passage 8 formed in a flow guide wall 7 of this centrifugal pump unit is different from that of the conventional precessing centrifugal pump.

In this example, the inlet passage 8 is formed so as to have an spiral opening extending from a top end portion d of the flow guide wall 7 through the whole length thereof (between the positions a to b shown in Fig. 6(a). The overall pumping performance can be thereby improved because, for example, the blood inflow area is increased so that the pumping effect of a rod 14 can also be utilized.

Figs. 7 and 8 show another example of the centrifugal pump unit of the precessing centrifugal pump of the present invention in which the pump outlet 5 has an improved structure. In the conventional pump, the outlet 5 extends tangentially from a portion of the side wall 2a of the casing 2 smoothly expanded outward. In contrast, in this example, an outer peripheral portion of the outlet 5 extending tangentially from the annular chamber 3 is slightly projected in a direction opposite to the flow whirling direction.

Various experiments have revealed that if the outlet is formed in this manner, occurrence of a vortex flow at this position can be prevented, which is caused by mixing of an outer peripheral flow having a larger whirling radius and high potential energy and an inner peripheral flow having lower potential energy, and that the flows are made uniform.

Preferably, the outlet 5 has the shape of a diffuser having a cross-sectional area gradually increased from the annular chamber 3 toward the outer end, and the diffuser spreading angle is set to a range of 7 to 8 degrees, thereby enabling the fluid to flowing smoothly from the annular chamber 3 to the outlet 5 while limiting the pressure loss.

Fig. 9 shows a still another example of the centrifugal pump unit of the present invention in which the impeller 6 has a club-like head 9. More specifically, the height of a conical base portion 9b of the impeller head 9 is increased to form a club-like shape.

If the impeller head 9 has such as club-like shape, the fluid flowing into the annular chamber 3 of the pump can be led to the outlet 5 while whirling energy is being gradually applied to the fluid, thereby improving the pumping efficiency.

Next, a motor unit II of a precessing centrifugal pump of the present invention will be described below.

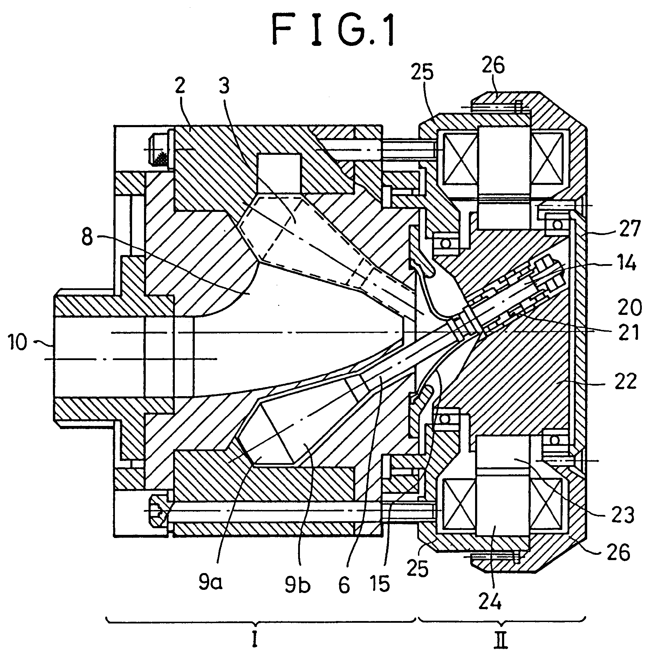

Fig. 1 shows a precessing centrifugal pump integrally combined with a motor in accordance with an embodiment of the present invention. A motor unit II is positioned adjacent to a centrifugal pump unit I and is integrally connected to the same.

The structure of the motor unit II is as described below.

The rod 14 of the impeller 6 is fixed to a circular rotary member 22 by a washer 20, a positioning nut 21 and so on.

A rotor 23 formed of a magnet rotated together with the rotary member 22 is attached to an outer circumferential portion of the rotary member 22. A coil 24 is disposed on the outer circumferential side of the rotor 23 so as to face the rotor 23. The rotary member 22, the rotor 23 and the coil 24 are retained and surrounded by holders 25 and 26 and a cover 27.

In the motor section II thus-constructed unlike the ordinary motor, the coil 24 is fixed while the rotor 23 formed of a magnet and the rotary member 22 attached to the rotor 23 are rotated. As the rotary member 22 is rotated, the rod 14 of the impeller 6 fixed to the rotary member 22 precesses.

Thus, the centrifugal pump unit I and the motor unit II are integrally combined to form a precessing centrifugal pump which has a simpler overall structure, which can be embedded in a human body, and which can be conveniently used as a pump-oxygenator blood pump.

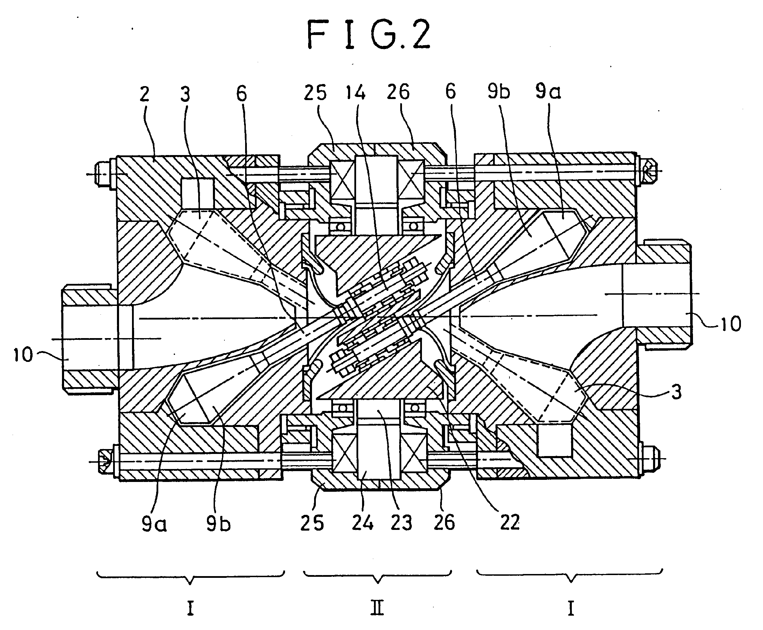

Fig. 2 shows a precessing centrifugal pump integrally combined with a motor in accordance with another embodiment of the present invention. This pump has a structure in which two pump units are connected to opposite sides of one motor unit.

In this embodiment, centrifugal pump units Ia and Ib are respectively attached to opposite side portions of a motor unit II. The two pump units can be driven by one motor. This embodiment is therefore advantageous in terms of space factor and energy utilization. In this case, the two pumps are rotated at the same speed.

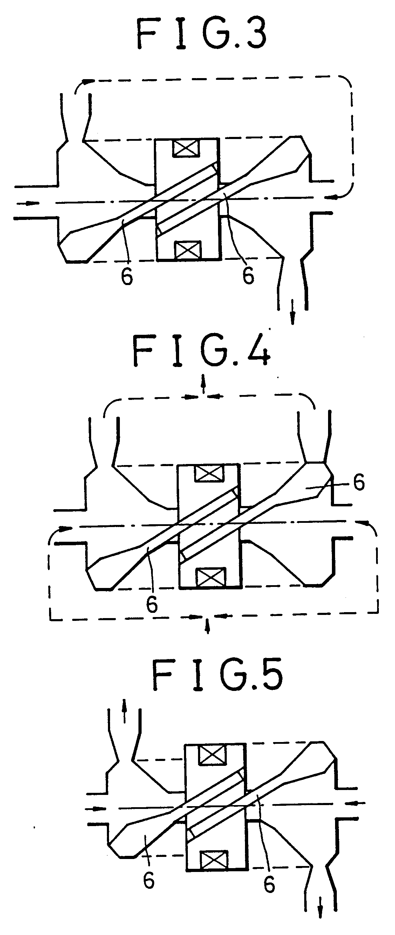

If the two pump units are equal in construction, they may be connected in series as shown in Fig. 3 to double the pumping characteristic pressure, or may be connected in parallel as shown in Fig. 4 to double the pumping characteristic flow rate.

Referring to Fig. 5, if the two pump units differ from each other in pumping characteristics, for example, if as in the case of a pump-oxygenator the flow rates for the right and left hearts are substantially equal but the pressure for the right heat is about 1/3 the pressure for the left heart, the radius of the impeller 6 of the centrifugal pump for the right heart may be reduced to 1/√3. In practice, the arrangement may be such that the radium of the impeller 6 is set to 1/1.5 and pressure adjustment is effected by throttling.

As described above, the precessing centrifugal pump in accordance with the present invention has a centrifugal pump unit and a motor unit integrally combined with each other, can therefore be directly embedded in a human body, and is very conveniently used for a pump-oxygenator. Moreover, the specific shape of the inlet passage ensures that the fluid can low smoothly and that the pumping efficiency can be improved.

The arrangement having two centrifugal pump units connected to opposite sides of a motor unit is advantageous in terms of space factor and energy utilization because two pump units can be operated by one motor.

## Claims

1. A precessing centrifugal pump comprising:
a centrifugal pump unit (I) including an annular chamber (3) formed in a generally conical casing (2) and defined by a side wall of the casing at the outer peripheral side, an impeller (6) having a head (9) and a rod (14) movably accommodated in the annular chamber, a flow guide wall (7) projecting from the center of a circular end surface of the casing into the annular chamber to define same at the inner peripheral side thereof, an inlet passage (8) formed in the flow guide wall, an inlet (10) communicating with the annular chamber through the inlet passage and opening at the center of the circular end surface of the casing, an outlet (5) extending tangentially from the annular chamber, the rod of the impeller extending through an opening of the casing formed at the apex thereof, and a sealing membrane (15) covering the opening of the casing and attached in a water-tight manner to a side wall of the casing and to the impeller, and a motor unit (II) including a rotary member (22) to which the rod of the impeller is attached and fixed,
characterized by the following features:
a) the inlet passage (8) being formed so as to have a spiral opening extending from a projecting end portion (d) of the flow guide wall (7) through the whole length thereof;
b) the motor unit (II) having a rotor (23) having a magnet attached to an outer circumferential surface of the rotary member and rotated together with the rotary member, and a coil (24) disposed so as to face the magnet; and
c) the motor unit (II) being integrally combined with said centrifugal pump unit (I).

2. A precessing centrifugal pump as claimed in Claim 1, wherein an outer periphery of said outlet (5) tangentially extending from said annular chamber (3) exhibits a smooth expansion which is convex in the direction reverse to that of the whirling of a flow.

3. A precessing centrifugal pump as claimed in Claim 1, wherein the head (9) of said impeller (6) has a club-like configuration.

## Patentansprüche

1. Präzessions-Zentrifugalpumpe mit:
einer Zentrifugalpumpeneinheit (I), die eine ringförmige Kammer (3) enthält, die in einem im wesentlichen konischen Gehäuse (2) ausgebildet ist und durch eine Seitenwand des Gehäuses an der äußeren Umfangsseite definiert ist, einem Flügel- bzw. Laufrad (6), das einen Kopf (9) und eine bewegbar in die ringförmige Kammer aufgenommene Stange bzw. Stab (14) hat, einer Strömungsführungswand (7), die von dem Zentrum einer kreisförmigen Endoberfläche des Gehäuses in die ringförmige Kammer vorsteht, um dieselbe an ihrer inneren Umfangsseite zu definieren, einen Einlaßdurchgang (8), der in der Strömungsführungswand ausgebildet ist, einen Einlaß (10), der über den Einlaßdurchgang mit der ringförmigen Kammer in Verbindung steht und sich an dem Zentrum der kreisförmigen Endoberfläche des Gehäuses öffnet, einem Auslaß (5), der sich tangential von der ringförmigen Kammer erstreckt, wobei sich die Stange bzw. der Stab des Flügelrades durch eine Öffnung in dem Gehäuse erstreckt, die an dessen Scheitelpunkt ausgebildet ist, und eine Dichtungsmembran (15), die die Öffnung des Gehäuses abdeckt und wasserdicht an eine Seitenwand des Gehäuses und an das Flügelrad angesetzt ist, und mit einer Motoreinheit (II), die ein rotierendes Element (22) enthält, an welchem der Stab bzw. die Stange des Flügelrades angesetzt und befestigt ist,
**gekennzeichnet** durch die folgenden Merkmale:
a) der Einlaßdurchgang (8) ist so ausgebildet, daß sich eine spiralförmige Öffnung von einem vorstehenden Endabschnitt (d) der Strömungsführungswand (7) über seine gesamte Länge erstreckt;
b) die Motoreinheit (II) hat einen Rotor (23) mit einem Magneten, der an einer äußeren Umfangsoberfläche des rotierenden Elements angebracht ist und zusammen mit dem rotierenden Element rotiert wird, und eine Spule bzw. Wicklung (24), die so angeordnet ist, daß sie dem Magneten zugewandt ist; und
c) die Motoreinheit (II) ist integral mit der Zentrifugalpumpeneinheit (I) kombiniert.

2. Präzessions-Zentrifugalpumpe nach Anspruch 1, wobei ein äußerer Umfang des Auslasses (5), der sich tangential von der ringförmigen Kammer (3) erstreckt, eine glatte bzw. gleichmäßig Expansion bzw. Aufweitung zeigt, welche konvex in der Richtung entgegengesetzt zu der der Wirbelbewegung einer Strömung ist.

3. Präzessions-Zentrifugalpumpe nach Anspruch 1, wobei der Kopf (9) des Flügelrades (6) eine knüppelartige Konfiguration aufweist.

## Revendications

1. Pompe centrifuge à précession, comprenant:
une unité de pompe centrifuge (I) comprenant une chambre annulaire (3) formée dans un boîtier de forme générale conique (2) et définie par une paroi latérale du boîtier sur le côté périphérique extérieur, un rotor (6) possédant une tête (9) et un arbre (14) étant logés à déplacement dans la chambre annulaire, une paroi (7) de guidage d'écoulement débordant depuis le centre d'une surface circulaire d'extrémité du boîtier dans la chambre annulaire, pour définir celle-ci sur son côté périphérique intérieur, un passage d'entrée (8) formé dans la paroi de guidage d'écoulement, une entrée (10) communicant avec la chambre annulaire à travers le passage d'entrée et s'ouvrant sur le centre de la surface circulaire d'extrémité du boîtier, une sortie (5) s'étendant tangentiellement depuis la chambre annulaire, l'arbre du rotor traversant une ouverture du boîtier formée au sommet de celui-ci, et une membrane d'étanchéité (15) couvrant l'ouverture du boîtier et étant fixée de manière étanche à l'eau à une paroi latérale du boitier et au rotor, et une unité de moteur (II) comprenant un membre tournant (22) auquel l'arbre du rotor est attache et fixé, caractérisée en ce que:
a) le passage d'entrée (8) est configuré de manière à posséder une ouverture en spirale s'étendant entre une partie d'extrémité en saillie (d) de la paroi (7) de guidage d'écoulement, sur toute la longueur de celle-ci;
b) l'unité de moteur (II) possède un rotor (23) ayant un aimant fixé à une surface périphérique extérieure du membre tournant et mis en rotation solidaire avec le membre tournant, et un bobinage (24) agencé de manière à faire face à l'aimant; et
c) l'unité de moteur (II) est intégrée à ladite unité de pompe centrifuge (I).

2. Pompe centrifuge à précession selon la revendication 1, dans laquelle une périphérie extérieure de la dite sortie (5) s'étend tangentiellement depuis ladite chambre annulaire et présente un relief adouci qui est convexe dans la direction opposée à celle du tourbillon d'un écoulement.

3. Pompe centrifuge à précession selon la revendication 1, dans laquelle la tête (9) dudit rotor (6) a une configuration ressemblant celle d'un bâton épaissi à une extrémité.
